# DEMANDE DE BREVET EUROPEEN

(11) **EP 1 589 466 A1**
(43) Date de publication de la demande: **26.10.2005**
(21) Numéro de dépôt: 05290895.1
(22) Date de dépôt: 22.04.2005
(51) Int. Cl.: G06F 19/00

(54) **Procédé et systeme de distribution de médicaments à des personnes dependantes**

(30) Priorité: 23.04.2004 FR 0404342
(71) Demandeur: Medissimo, 78306 Poissy Cedex (FR)
(72) Inventeur: Blochet, Caroline, 78100 Saint Germain en Laye (FR)
(74) Mandataire: Pichat, Thierry

(57) **Abrégé**

Pour la délivrance de produits réglementés à des personnes dépendantes, ce procédé comprenant des étapes au cours desquelles : des informations d'ordonnances (5) sont introduites dans une base de données (4) connectée à un ordinateur (1) ; l'ordinateur génère une liste de boîtes de produits (6) couvrant les besoins définis par les informations d'ordonnances ; l'ordinateur génère pour chaque boîte de produits figurant dans la liste, un numéro de suivi unique respectif, et commande l'impression d'étiquettes (7) à apposer sur les boîtes de produits, sur lesquelles figurent respectivement les numéros de suivi générés pour chaque boîte de produit figurant dans la liste ; le numéro de suivi imprimé sur l'étiquette une fois apposée sur la boîte et des informations d'identification relatives au produit indiquées sur la boîte de produit sont introduits en association dans la base de données; et les produits figurant dans la liste sont regroupés par patient en vue de leur distribution.

## Description

La présente invention concerne la dispensation de médicaments à des personnes dépendantes hébergées en établissement médico-social tel qu'une maison de retraite ou une maison pour personnes handicapées.

Elle s'applique plus généralement à la distribution de produits de santé réglementés.

La dispensation d'un médicament est un acte pharmaceutique qui conduit en principe à la délivrance du médicament au patient à qui il est administré. La délivrance d'un médicament est la remise matérielle de celui-ci par une personne habilitée.

Habituellement, la dispensation de médicaments dans les établissements médico-sociaux qui hébergent des personnes dépendantes est effectuée suivant la procédure suivante. Une personne habilitée de l'établissement collecte les ordonnances des patients pour les envoyer à une pharmacie. La pharmacie prépare un paquet de médicaments pour chaque ordonnance reçue et livre les paquets à l'établissement. Une infirmière n'ayant a priori pas la qualification de pharmacien est ensuite chargée d'administrer les médicaments aux patients concernés en fonction des informations de prescription figurant dans les ordonnances.

Or, la préparation et l'administration de doses de médicaments en établissement médico-social par une personne non qualifiée porte atteinte au monopole pharmaceutique qui inscrit l'acte de délivrance d'un médicament comme l'aboutissement de l'acte de dispensation réalisé sous la responsabilité du pharmacien. Si une personne non qualifiée intervient pour administrer les médicaments aux patients, l'acte du pharmacien se trouve réduit à celui d'un distributeur de médicaments, et l'administration des doses de médicaments n'est plus effectuée sous la responsabilité du pharmacien, ce qui tend à mettre le patient en danger.

Par ailleurs, en cas d'alerte sur un médicament émise par un laboratoire pharmaceutique, il n'est pas possible aujourd'hui de déterminer rapidement à quels patients a été distribué un médicament ayant un numéro de lot déterminé, afin qu'il ne soit plus administré.

La présente invention a pour but de supprimer ces inconvénients en proposant un procédé permettant d'assister techniquement le pharmacien dans la préparation et la délivrance de médicaments à un patient dépendant, tout en offrant au patient une sécurité comparable à celle dont il bénéficierait si le pharmacien lui remettait directement les médicaments. Cet objectif est atteint par la prévision d'un procédé de délivrance de produits dont la distribution est réglementée à des personnes dépendantes, ce procédé comprenant des étapes au cours desquelles :
- des informations d'ordonnances introduites sont mémorisées dans une base de données stockée par un ordinateur, les informations d'ordonnances comprenant pour chaque ordonnance des informations d'identification de patient, des identifiants de produits, et pour chaque identifiant de produit, une durée de traitement, un nombre de prises journalières et une quantité de produit par prise journalière,
- l'ordinateur génère à partir des informations d'ordonnances introduites une liste de boîtes de produits couvrant les besoins définis par les informations d'ordonnances.

Selon l'invention, ce procédé comprend en outre des étapes au cours desquelles :
- a) l'ordinateur génère pour chaque boîte de produits figurant dans la liste de boîtes de produits, un numéro de suivi unique respectif, et commande l'impression d'étiquettes à apposer sur les boîtes de produits et sur lesquelles figurent respectivement les numéros de suivi générés,
- b) pour chaque boîte de produit figurant dans la liste, le numéro de suivi imprimé sur l'étiquette une fois apposée sur la boîte et des informations d'identification relatives au produit indiquées sur la boîte de produit sont introduits et mémorisés en association dans la base de données, et
- c) les produits figurant dans la liste sont regroupés par patient en vue de leur distribution aux patients correspondants.

Ainsi, il est prévu une étape consistant à apposer sur les boîtes de produits les étiquettes imprimées. Et, lors de l'étape c), ce sont donc les boîtes desdits produits, avec leurs étiquettes apposées, qui sont regroupées par patient.

Selon un mode de réalisation de l'invention, ce procédé comprend en outre des étapes au cours desquelles :
- pour chaque ordonnance et chaque produit se présentant sous la forme de pilules, mentionné dans l'ordonnance, les pilules dont le nombre correspond à la durée de traitement, le nombre de prises journalières et la quantité de produit par prise journalière, sont déconditionnées et reconditionnées dans au moins un emballage,
- l'emballage est refermé hermétiquement, et
- l'ordinateur commande l'impression d'une étiquette destinée à être collée sur chaque emballage refermé sur laquelle figurent des informations d'identification de patient, des informations d'identification relatives au produit, et des informations relatives à l'heure de la prise du produit.

Selon un mode de réalisation de l'invention, les boîtes de produits se présentant sous la forme de pilules contenant les pilules non reconditionnées sont regroupées en vue de leur archivage, et l'ordinateur commande l'impression d'une étiquette d'archivage mentionnant les numéros de suivi apposés sur les boîtes ainsi archivées.

De préférence, les emballages comprennent des alvéoles destinées à recevoir respectivement une prise journalière d'un produit.

Selon un mode de réalisation de l'invention, le numéro de suivi est imprimé sur les étiquettes sous la forme d'un code à barres qui est lu pour être introduit dans la base de données à l'aide d'un lecteur de codes à barres.

Selon un mode de réalisation de l'invention, à la suite d'une alerte émise pour un produit, contenant des informations d'identification d'un produit à retirer, l'ordinateur recherche dans la base de données les informations relatives au produit et aux patients auxquels a été distribué le produit à retirer, et génère un ordre de récupération contenant les informations d'identification du produit à retirer et une liste d'informations d'identification des patients auxquels a été distribué le produit.

Selon un mode de réalisation de l'invention, les informations d'identification de chaque patient comprennent des informations d'identification d'établissement hébergeant le patient, le procédé comprenant une étape au cours de laquelle l'ordinateur prépare et imprime un bordereau de livraison pour chaque établissement hébergeant des patients auxquels des produits sont à distribuer.

L'invention concerne également un système de délivrance de produits dont la distribution est réglementée à des personnes dépendantes, ce système comprenant un ordinateur connecté à une base de données, des moyens pour introduire dans la base de données des informations d'ordonnances comprenant pour chaque ordonnance des informations d'identification de patient, des identifiants de produits, et pour chaque identifiant de produit, une durée de traitement, un nombre de prises journalières et une quantité de produit par prise journalière, et des moyens pour générer à partir des informations d'ordonnances introduites une liste de boîtes de produits couvrant les besoins définis par les informations d'ordonnances.

Selon l'invention, l'ordinateur comprend en outre :
- des moyens pour générer pour chaque boîte de produit figurant dans la liste de boîtes de produits, un numéro de suivi unique respectif, et pour commander l'impression d'étiquettes à apposer sur les boîtes de produits et sur lesquelles figurent respectivement les numéros de suivi générés, et
- des moyens pour introduire en association dans la base de données, pour chaque boîte de produit figurant dans la liste, le numéro de suivi imprimé sur l'étiquette une fois apposée sur la boîte de produit, et des informations d'identification relatives au produit indiquées sur la boîte de produit.

Selon un mode de réalisation de l'invention, l'ordinateur comprend en outre des moyens pour commander, pour chaque produit spécifié dans une ordonnance, chaque prise journalière du produit, l'impression d'une étiquette sur laquelle figurent des informations d'identification de patient, des informations d'identification relatives à produit prescrit au patient, et des informations relatives à l'heure de la prise du produit.

Selon un mode de réalisation de l'invention, l'ordinateur comprend en outre des moyens pour commander l'impression d'une étiquette d'archivage mentionnant les numéros de suivi apposés sur des boîtes de produit figurant dans la liste de boîtes de produits et déconditionnées.

Selon un mode de réalisation de l'invention, ce système comprend des moyens de lecture de codes à barres, l'ordinateur comprenant des moyens pour imprimer les numéros de suivi sous la forme de codes à barres.

Selon un mode de réalisation de l'invention, l'ordinateur comprend des moyens pour rechercher dans la base de données les informations relatives à un produit à retirer et aux patients auxquels a été distribué le produit à retirer, et des moyens pour générer un ordre de récupération contenant les informations d'identification du produit à retirer et une liste d'informations d'identification des patients auxquels a été distribué le produit.

Un mode de réalisation préféré de l'invention sera décrit ci-après, à titre d'exemple non limitatif, avec référence aux dessins annexés dans lesquels :
La figure 1 représente schématiquement un système mis en oeuvre par le procédé selon l'invention pour distribuer des médicaments à des personnes dépendantes ;
La figure 2 illustre schématiquement le procédé selon l'invention.

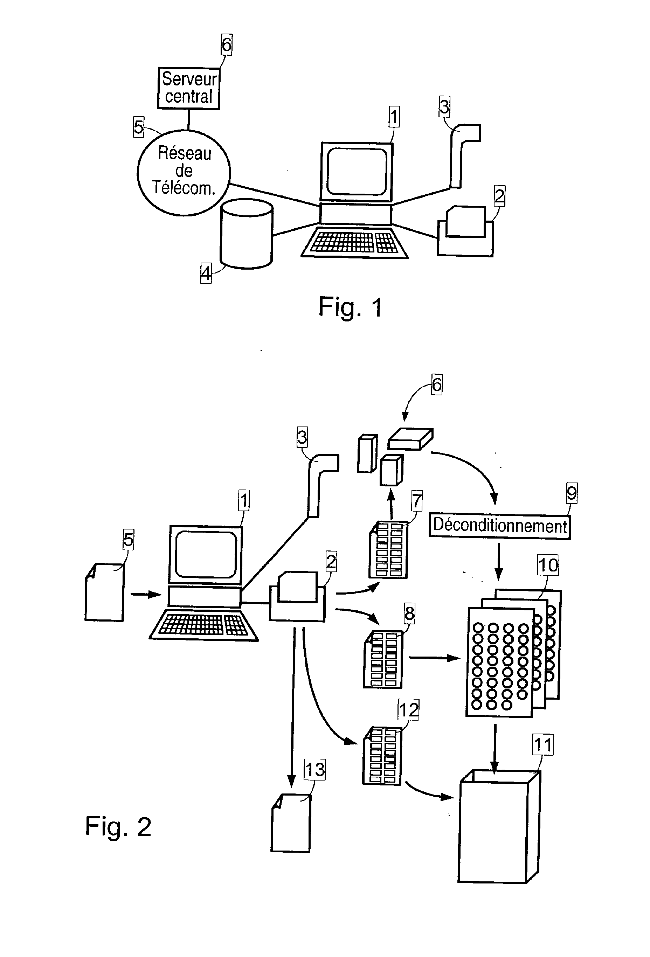

Le système de distribution représenté sur la figure 1 comprend un ordinateur 1, par exemple de type PC installé dans une pharmacie, connecté à une imprimante 2, à un lecteur de codes à barres 3 et à une base de données 4.

L'ordinateur 1 est équipé d'un logiciel pharmaceutique classique permettant notamment la saisie des médicaments, l'impression de feuilles de remboursement et la gestion du stock de médicaments de la pharmacie.

En outre, l'ordinateur 1 est équipé d'un logiciel spécifique selon l'invention permettant la traçabilité des médicaments de la pharmacie jusqu'à leur administration aux patients hébergés dans un établissement médico-social.

Conformément au procédé selon l'invention illustré sur la figure 2, les ordonnances 5 reçues de l'établissement médico-social sont saisies sur le logiciel pharmaceutique qui imprime également les feuilles de soin correspondantes. La saisie des ordonnances consiste à saisir les informations d'identification du patient, du médecin qui a émis l'ordonnance, et des médicaments prescrits, ainsi que la quantité de médicament (nombre de boîtes) à retirer du stock.

Si ces informations ne sont pas déjà saisies, un opérateur introduit en outre dans la base de données 4 pour chaque médicament prescrit et pour chaque patient, la durée en nombre de jours du traitement, le nombre de prises journalières et le nombre de doses de médicament à administrer à chaque prise.

Le nombre de boîtes par médicament à retirer du stock est avantageusement déterminé automatiquement par le logiciel à partir des informations de posologie prescrites dans l'ordonnance (nombre de prises journalières et nombre de doses de médicament pour chaque prise) et de durée de traitement pour chaque médicament, ainsi que du nombre de doses par boîte pour chaque médicament, cette dernière information étant fournie par exemple par la base de données 4.

Le logiciel de traçabilité intercepte les données d'ordonnance saisies et établit une liste récapitulative de déstockage de médicaments, présentant les identifiants des médicaments et par médicament la quantité (nombre de boîtes) à retirer du stock de la pharmacie. Egalement à partir de ces données d'ordonnance, il insère dans la base de données 4 les informations d'identification des nouveaux patients éventuels.

Des informations complémentaires sur chaque patient telles que le nom de l'établissement où il est hébergé, et son numéro de chambre peuvent en outre être introduites par un opérateur dans la base de données 4.

L'opérateur retire ensuite du stock de la pharmacie les boîtes de médicament 6 telles que spécifiées dans la liste récapitulative de déstockage, et déclenche à l'aide du logiciel l'impression d'étiquettes 7 à coller sur les boîtes extraites. Ces étiquettes comportent de préférence sous la forme d'un code à barres un code de suivi unique qui est généré consécutivement par le logiciel et qui intègre un identifiant de la pharmacie, de sorte que deux boîtes de médicaments auront deux numéros de suivi différents. L'opérateur colle alors les étiquettes imprimées 7 sur les boîtes de médicament 6.

Vient ensuite une phase de déconditionnement des médicaments sous blister, au cours de laquelle, le logiciel de traçabilité lit séquentiellement chaque ordonnance, telle qu'enregistrée dans la base de données 4, et pour chaque ordonnance lue, et chaque médicament mentionné dans l'ordonnance, il demande à l'opérateur de présenter une boîte du médicament devant le lecteur de codes à barres 3 de manière à lire le code de suivi, et si celui-ci existe, le code à barres apposé par le fabriquant sur la boîte et donnant le code d'identification CIP (Club Inter Pharmaceutique), le numéro de lot et la date de péremption du médicament. Si ce dernier code à barres ne figure pas sur la boîte de médicament, l'opérateur doit saisir manuellement ces informations. Les informations lues sur la boîte ou saisies par l'opérateur sont ensuite enregistrées dans la base de données 4 en association avec le code de suivi apposé sur la boîte.

L'association de codes de suivi respectifs aux boîtes de médicament retirées du stock permet d'éviter les erreurs de manipulation et de suivre un médicament depuis le stock de la pharmacie jusqu'à son administration au patient. En effet, la sécurité dans le suivi d'un médicament réside dans la lecture simultanée du code de suivi apposé sur la boîte de médicament et du code d'identification et du numéro de lot du médicament.

S'il s'agit d'un médicament en comprimés, pilules ou gélules, le logiciel de traçabilité demande à l'opérateur de déconditionner le médicament et de placer chaque comprimé dans une alvéole respective d'une plaquette 10 comportant par exemple 31 alvéoles (pour un mois de traitement). Une fois qu'une plaquette est ainsi remplie, l'opérateur procède à sa fermeture hermétique, par exemple par thermo-scellement d'un film.
Le logiciel prépare ensuite l'impression d'une étiquette 8 sur laquelle figurent notamment les informations suivantes :
- un numéro de plaquette unique intégrant un code d'identification de la pharmacie,
- le nom du patient,
- le nom de l'établissement où il est hébergé,
- le numéro de sa chambre,
- la date de déconditionnement (date du jour),
- le nom du médicament,
- le numéro de lot du médicament,
- la date de péremption du médicament, et
- l'heure de la prise du médicament.

L'opérateur appose ensuite sur la plaquette 10 l'étiquette 8 qui a été imprimée pour cette prise de médicament.

Les numéros de plaquettes qui sont générés par le logiciel au moment de l'impression des étiquettes 8 sont également mémorisés dans la base de données 4.

L'opérateur prépare ainsi une plaquette 10 pour chaque médicament, chaque prise journalière de ce médicament. Par exemple, pour une posologie de trois comprimés d'un médicament à prendre trois fois par jour, l'opérateur produira ainsi pour au plus un mois de traitement trois plaquettes dans lesquelles chaque alvéole renferme trois comprimés. Parallèlement, le logiciel déclenche l'impression d'autant d'étiquettes 8 pour un médicament que de plaquettes à produire.

De cette manière, seule la quantité de comprimés prescrite dans l'ordonnance est reconditionnée, les comprimés restants étant replacés dans la boîte du médicament pour archivage.

Tous les médicaments qui ne sont pas en comprimés et les plaquettes 10 ainsi préparés pour un patient sont insérés dans un paquet de livraison 11 unique pour chaque patient. L'opérateur peut également disposer dans le paquet, l'ordonnance du patient et les notices de chaque médicament prescrit sur l'ordonnance. Le paquet est ensuite scellé en fin de préparation de l'ordonnance. Le logiciel déclenche ensuite l'impression d'une étiquette 12 mentionnant notamment le nom du patient, le nom de l'établissement où il est hébergé, le numéro de sa chambre et la date de conditionnement, cette étiquette étant apposée sur le paquet par l'opérateur.

Au fur et à mesure du traitement des ordonnances, on peut prévoir que le logiciel vérifie la date de péremption de chaque lot de médicament par rapport à la date courante de conditionnement, et génère une alerte si par exemple la date de péremption est inférieure de moins de trois mois à la date courante.

A la fin du traitement de toutes les ordonnances d'un établissement, le logiciel prépare l'impression d'un bordereau de livraison 13 récapitulant les médicaments émis pour l'établissement et par patient, les noms des médicaments n'étant pas mentionnés dans un souci de sécurité du transport. Les paquets 11 de livraison de médicaments pour chaque patient sont ensuite livrés à l'établissement avec le bordereau de livraison.

Par exemple en fin de journée, le logiciel procède également à l'archivage des boîtes de médicaments. A cet effet, il génère une étiquette indiquant la liste des numéros de suivi générés pour ces médicaments (par exemple les premier et dernier numéros de suivi générés pour ces boîtes), cette étiquette étant destinée à être apposée sur l'emballage dans lequel sont insérées ces boîtes. Les boîtes de médicaments sont ainsi conservées un certain temps, ce qui permet au pharmacien de reproduire les informations fournies par le fabriquant et apposées sur les boîtes de médicament.

Les boîtes de médicament ainsi archivées peuvent également être transmises à l'établissement médico-social sur demande expresse de ce dernier.

Le logiciel est en outre conçu pour générer des listes de plaquettes 10 à récupérer en cas de dépassement de la date de péremption.

Par ailleurs, le logiciel est conçu pour générer un ordre de récupération à partir d'un code d'identification de médicament et d'un numéro de lot, afin de permettre, en cas de nécessité de retirer un lot de médicaments. A cet effet, le logiciel est conçu pour éditer à la demande des ordres de récupération à raison d'un ordre de récupération par établissement médico-social contenant l'identifiant du médicament (code CIP et numéro de lot) et/ou les numéros des plaquettes 10 à récupérer qui sont obtenues par une requête à la base de données 4 à partir de l'identifiant du médicament à récupérer et du numéro de lot.

De préférence, l'ordinateur 1 est relié par l'intermédiaire d'un réseau de télécommunication 5 à un serveur central 6, de manière à transmettre périodiquement toutes les données relatives aux médicaments délivrés à l'exception des données permettant d'identifier les patients et les médecins prescripteurs. Ces données sont utilisées par le serveur central dans un but de sécurisation de la traçabilité, et afin d'assurer une meilleure gestion des alertes et de permettre d'établir des statistiques sur les prescriptions de médicaments.

## Revendications

1. Procédé de délivrance de produits dont la distribution est réglementée à des personnes dépendantes, ce procédé comprenant des étapes au cours desquelles :
- des informations d'ordonnances (5) introduites sont mémorisées dans une base de données (4) stockée par un ordinateur (1), les informations d'ordonnances comprenant pour chaque ordonnance des informations d'identification de patient, des identifiants de produits, et pour chaque identifiant de produit, une durée de traitement, un nombre de prises journalières et une quantité de produit par prise journalière,
- l'ordinateur génère à partir des informations d'ordonnances introduites une liste de boîtes de produits (6) couvrant les besoins définis par les informations d'ordonnances,
**caractérisé en ce qu'**il comprend en outre des étapes au cours desquelles :
- l'ordinateur génère pour chaque boîte de produits (6) figurant dans la liste de boîtes de produits, un numéro de suivi unique respectif, et commande l'impression d'étiquettes (7) à apposer sur les boîtes de produits et sur lesquelles figurent respectivement les numéros de suivi générés,
- pour chaque boîte de produit figurant dans la liste, le numéro de suivi imprimé sur l'étiquette une fois apposée sur la boîte et des informations d'identification relatives au produit indiquées sur la boîte de produit sont introduits et mémorisés en association dans la base de données (4), et
- les produits figurant dans la liste sont regroupés par patient en vue de leur distribution aux patients correspondants.

2. Procédé selon la revendication 1,
**caractérisé en ce qu'**il comprend en outre des étapes au cours desquelles :
- pour chaque ordonnance (5) et chaque produit se présentant sous la forme de pilules, mentionné dans l'ordonnance, les pilules dont le nombre correspond à la durée de traitement, le nombre de prises journalières et la quantité de produit par prise journalière, sont déconditionnées et reconditionnées dans au moins un emballage (10),
- l'emballage est refermé hermétiquement, et
- l'ordinateur (1) commande l'impression d'une étiquette (8) destinée à être collée sur chaque emballage refermé sur laquelle figurent des informations d'identification de patient, des informations d'identification relatives au produit, et des informations relatives à l'heure de la prise du produit.

3. Procédé selon la revendication 2,
**caractérisé en ce que** les boîtes de produits (6) se présentant sous la forme de pilules contenant les pilules non reconditionnées sont regroupées en vue de leur archivage, et l'ordinateur (1) commande l'impression d'une étiquette d'archivage mentionnant les numéros de suivi apposés sur les boîtes ainsi archivées.

4. Procédé selon la revendication 2 ou 3,
**caractérisé en ce que** les emballages (10) comprennent des alvéoles destinées à recevoir respectivement une prise journalière d'un produit.

5. Procédé selon l'une des revendications 1 à 4,
**caractérisé en ce que** le numéro de suivi est imprimé sur les étiquettes (7) sous la forme d'un code à barres qui est lu pour être introduit dans la base de données (4) à l'aide d'un lecteur (3) de codes à barres.

6. Procédé selon l'une des revendications 1 à 5,
**caractérisé en ce qu'**à la suite d'une alerte émise pour un produit, contenant des informations d'identification d'un produit à retirer, l'ordinateur (1) recherche dans la base de données (4) les informations relatives au produit et aux patients auxquels a été distribué le produit à retirer, et génère un ordre de récupération contenant les informations d'identification du produit à retirer et une liste d'informations d'identification des patients auxquels a été distribué le produit.

7. Procédé selon l'une des revendications 1 à 6,
**caractérisé en ce que** les informations d'identification de chaque patient comprennent des informations d'identification d'établissement hébergeant le patient, le procédé comprenant une étape au cours de laquelle l'ordinateur (1) prépare et imprime un bordereau de livraison pour chaque établissement hébergeant des patients auxquels des produits sont à distribuer.

8. Système de délivrance de produits dont la distribution est réglementée à des personnes dépendantes, ce système comprenant un ordinateur (1) connecté à une base de données (4), des moyens pour introduire dans la base de données des informations d'ordonnances comprenant pour chaque ordonnance des informations d'identification de patient, des identifiants de produits, et pour chaque identifiant de produit, une durée de traitement, un nombre de prises journalières et une quantité de produit par prise journalière, et des moyens pour générer à partir des informations d'ordonnances introduites une liste de boîtes de produits (6) couvrant les besoins définis par les informations d'ordonnances,
**caractérisé en ce que** l'ordinateur (1) comprend en outre :
- des moyens pour générer pour chaque boîte de produit (6) figurant dans la liste de boîtes de produits, un numéro de suivi unique respectif, et pour commander l'impression d'étiquettes (7) à apposer sur les boîtes de produits (6) et sur lesquelles figurent respectivement les numéros de suivi générés, et
- des moyens pour introduire en association dans la base de données, pour chaque boîte de produit figurant dans la liste, le numéro de suivi imprimé sur l'étiquette une fois apposée sur la boîte de produit, et des informations d'identification relatives au produit indiquées sur la boîte de produit.

9. Système selon la revendication 8,
**caractérisé en ce que** l'ordinateur (1) comprend en outre des moyens pour commander, pour chaque produit spécifié dans une ordonnance, chaque prise journalière du produit, l'impression d'une étiquette (8) sur laquelle figurent des informations d'identification de patient, des informations d'identification relatives à produit prescrit au patient, et des informations relatives à l'heure de la prise du produit.

10. Système selon la revendication 8 ou 9,
**caractérisé en ce que** l'ordinateur (1) comprend en outre des moyens pour commander l'impression d'une étiquette d'archivage mentionnant les numéros de suivi apposés sur des boîtes de produit figurant dans la liste de boîtes de produits et déconditionnées.

11. Système selon l'une des revendications 8 à 10,
**caractérisé en ce qu'**il comprend des moyens de lecture (3) de codes à barres, l'ordinateur (1) comprenant des moyens pour imprimer les numéros de suivi sous la forme de codes à barres.

12. Système selon l'une des revendications 8 à 11,
**caractérisé en ce que** l'ordinateur (1) comprend des moyens pour rechercher dans la base de données (4) les informations relatives à un produit à retirer et aux patients auxquels a été distribué le produit à retirer, et des moyens pour générer un ordre de récupération contenant les informations d'identification du produit à retirer et une liste d'informations d'identification des patients auxquels a été distribué le produit.
